# EUROPEAN PATENT APPLICATION

(11) **EP 4 403 180 A1**
(43) Date of publication of application: **24.07.2024**
(21) Application number: 22870257.7
(22) Date of filing: 14.09.2022
(51) Int. Cl.: A61K 36/87, A61K 47/12, A61K 47/26, A61P 31/04, A61P 17/10, A61P 39/06, A61K 8/9789, A61Q 17/00, A61Q 19/00, A23L 33/105

(54) **ANTIBACTERIAL AND ANTIOXIDANT COMPOSITION CONTAINING GRAPE CALLUS CULTURE MEDIUM AS ACTIVE INGREDIENT**

(30) Priority: 14.09.2021 KR 20210122731
(71) Applicant: Ingr Incorporated, Yongin-si, Gyeonggi-do 16890 (KR)
(72) Inventor: PYEE, Jae Ho, Gunpo-si, Gyeonggi-do 15804 (KR)
(74) Representative: Vossius & Partner Patentanwälte Rechtsanwälte mbB
(86) International application number: PCT/KR2022/013697
(87) International publication number: WO 2023/043173

(57) **Abstract**

The present invention relates to an antibacterial and antioxidant composition containing grape callus culture solution, as an active ingredient, comprising epsilon(ε)-viniferin, delta (δ)-viniferin, resveratrol, and the like, and, specifically, grape callus, its culture solution, and its extract according to the present invention exhibit no cytotoxicity, have significant antioxidant and antibacterial effect against *Propionibacterium acnes,* and have remarkable antioxidant and antibacterial effect compared to pure viniferin, in particular, and thus, the grape callus, its culture solution, and its extract can be effectively used as active ingredients in antioxidant and antibacterial compositions.

## Description

### Technical Field

The present invention relates to an antibacterial and antioxidant composition containing a grape callus culture solution containing effective ingredients such as epsilon(ε)-viniferin, delta(δ)-viniferin and resveratrol.

### Background Art

In the process of transitioning from an industrial-oriented society to a more industrial-centered society to a more eco-centered one globally, human health has become a top priority. While the rapid expansion of industrial society based on petroleum-based materials has brought convenience to humans, it has also resulted in environmental pollution and situations that harm human health due to the aftermath of rapid development. As a result, there is increasing interest and demand for environmentally friendly substances, particularly in products closely related to daily life.

Oxygen is an essential element for maintaining life, as it is used to produce energy through the respiratory process in the human body. However, oxygen not only provides energy to us but also generates harmful oxygen called reactive oxygen species (ROS) during the process of combining with food in the body. These ROS act as a cause of disease in our bodies, and recently, the medical community has identified them as the main culprit of human aging. Consequently, there is continuous development of natural antioxidant agents derived from substances that can protect the human body from the toxicity of ROS.

Acne is a skin condition that forms comedones or pustules and occurs in most people of all ages. The causes of acne are complex and include factors such as excessive sebum secretion, hormonal imbalance, follicular hyperkeratinization, proliferation of *Propionibacterium acnes,* and inflammatory responses. The acne-causing bacterium *P. acnes* proliferates in anaerobic conditions within the hair follicles, where it hydrolyzes triglycerides in sebum to produce free fatty acids, which stimulate the follicles chemotactically and trigger inflammatory responses. While antibiotic therapy is commonly used to treat acne caused by P. acnes infection, its efficacy may be compromised due to antibiotic resistance. Therefore, research into herbal-derived substances with no side effects and resistance in acne treatment is actively underway.

Meanwhile, there has been significant progress in biotechnological methods to produce various beneficial plant-derived compounds, such as secondary metabolites. Among these methods, plant cell culture is considered the most effective. This method can efficiently produce secondary metabolites through the manipulation of culture media, culture conditions, and various elicitors. Furthermore, it is commercially significant as it allows for the continuous and stable mass production of plant-derived beneficial substances without the constraints of cultivation or natural environmental limitations (such as toxicity of pesticides, heavy metal contamination, seasonal variations, climate conditions, etc.).

In addition, as a related prior document, Korean Patent No. 10-1253374 (cosmetic composition for acne skin improvement) uses Kimchi lactobacillus fermentation extract and soybean fermentation extract as cosmetic compositions for improving acne skin, and Korean Patent No. 10-0879032 (antibacterial, antioxidant and anti-inflammatory inhibitors having natural antibacterial and anti-inflammatory effects, and cosmetic compositions containing the same) provides a cosmetic composition having an antibacterial effect using mixed extracts of the herbal medicine such as *Glycyrrhiza uralensis* Fisch. *Anemarrhena asphodeloides* Bunge, *Aralia continentalis, Phellodendron amurense* Ruprecht, *Coptis japonica* Makino, *Sophora angustifolia* Sieb. et Zucc., *Scutellaria baicalensis* Georg., *Sophora japonica* L., *Lithospermum erythrorhizon* Sieb. et Zucc, and the antibacterial and antioxidant effects of grape callus on acne bacteria are unknown.

The inventors of the present invention have endeavored to establish a method for mass-producing plant-derived beneficial substances and have made efforts to develop functional cosmetics, health functional foods, and pharmaceutical materials using them, as a result, the grape callus culture solution of the present invention exhibits significant antioxidant and antibacterial effects, particularly shows markedly superior antioxidant and antibacterial effects compared to pure viniferin, and so, by demonstrating that the grape callus culture solution can be effectively used as a natural-derived antioxidant and antibacterial pharmaceutical composition, cosmetic composition, and health food, the present invention is completed.

The purpose of the present invention is to provide an antioxidant and antibacterial composition containing one or more selected from a group consisting of grape callus, its lysate, its culture solution, and its extract as active ingredients.

Furthermore, another object of the present invention is to provide a method for preventing, improving, or treating oxidative stress by administering a composition containing a pharmaceutically effective amount of one or more selected from a group consisting of grape callus, its lysate, its culture solution and its extract to a subject.

Additionally, another object of the present invention is to provide a method for preventing, improving, or treating bacterial infections by administering a composition containing a pharmaceutically effective amount of one or more selected from a group consisting of grape callus, its lysate, its culture solution and its extract to a subject.

Furthermore, another object of the present invention is to provide a method for preventing, improving, or treating acne by administering a composition containing a pharmaceutically effective amount of one or more selected from a group consisting of grape callus, its lysate, its culture solution and its extract to a subject.

Additionally, another object of the present invention is to provide a use for a composition containing one or more selected from a group consisting of grape callus, its lysate, its culture solution and its extract as active ingredients, for the prevention, improvement, or treatment of oxidative stress.

Moreover, another object of the present invention is to provide a use for a composition containing one or more selected from a group consisting of grape callus, its lysate, its culture solution and its extract as active ingredients, for the prevention, improvement, or treatment of bacterial infections.

Furthermore, another object of the present invention is to provide a use for a composition containing one or more selected from a group consisting of grape callus, its lysate, its culture solution and its extract as active ingredients, for the prevention or treatment of acne as a pharmaceutical composition.

Furthermore, another object of the present invention is to provide a use for a composition containing one or more selected from a group consisting of grape callus, its lysate, its culture solution and its extract as active ingredients, for the prevention and improvement of acne as a cosmetic composition.

To achieve the objectives of the present invention, the present invention provides a pharmaceutical composition for antioxidant and antibacterial purposes containing one or more selected from a group consisting of grape callus, its lysate, its culture solution and its extract as active ingredients.

Additionally, the present invention provides a pharmaceutical composition for the prevention and treatment of acne containing one or more selected from a group consisting of grape callus, its lysate, its culture solution and its extract as active ingredients.

Moreover, the present invention provides a cosmetic composition for antioxidant and antibacterial purposes containing one or more selected from a group consisting of grape callus, its lysate, its culture solution and its extract as active ingredients.

Furthermore, the present invention provides a cosmetic composition for the prevention and improvement of acne containing one or more selected from a group consisting of grape callus, its lysate, its culture solution and its extract as active ingredients.

Additionally, the present invention provides a health food for antioxidant and antibacterial purposes containing one or more selected from a group consisting of grape callus, its lysate, its culture solution and its extract as active ingredients.

Furthermore, the present invention provides a method for preventing or improving oxidative stress by administering a composition containing a pharmaceutically effective amount of one or more selected from a group consisting of grape callus, its lysate, its culture solution and its extract as active ingredients to a subject.

Additionally, the present invention provides a method for treating oxidative stress by administering a composition containing a pharmaceutically effective amount of one or more selected from a group consisting of grape callus, its lysate, its culture solution and its extract as active ingredients to a subject.

Furthermore, another object of the present invention is to provide a method for preventing or improving bacterial infections by administering a composition containing a pharmaceutically effective amount of one or more selected from a group consisting of grape callus, its lysate, its culture solution and its extract as active ingredients to a subject.

Additionally, another object of the present invention is to provide a method for treating bacterial infections by administering a composition containing a pharmaceutically effective amount of one or more selected from a group consisting of grape callus, its lysate, its culture solution and its extract as active ingredients to a subject.

Moreover, the present invention provides a method for preventing or improving acne by administering a composition containing a pharmaceutically effective amount of one or more selected from a group consisting of grape callus, its lysate, its culture solution and its extract as active ingredients to a subject.

Furthermore, the present invention provides a method for treating acne, comprising administering a composition containing one or more selected from a group consisting of grape callus, its lysate, its culture solution and its extract as active ingredients to a subject.

Additionally, the present invention provides a use of a composition for the prevention, improvement, or treatment of oxidative stress, comprising one or more selected from a group consisting of grape callus, its lysate, its culture solution and its extract as active ingredients.

Moreover, the present invention provides a use of a composition for the prevention, improvement, or treatment of bacterial infections, comprising one or more selected from a group consisting of grape callus, its lysate, its culture solution and its extract as active ingredients.

Furthermore, the present invention provides a use of a composition for the prevention or treatment of acne as a pharmaceutical composition, comprising one or more selected from a group consisting of grape callus, its lysate, its culture solution and its extract as active ingredients.

Additionally, the present invention provides a use of a composition for the prevention and improvement of acne as a cosmetic composition, comprising one or more selected from a group consisting of grape callus, its lysate, its culture solution and its extract as active ingredients.

The grape callus, its culture solution, and its extract of the present invention exhibit no cytotoxicity and demonstrate significant antioxidant and antibacterial effects against acne bacteria, particularly, compared to pure viniferin, they exhibit remarkable antioxidant and antibacterial effects, and therefore, the grape callus, its culture solution, and its extract can be effectively used as active ingredients in antioxidant and antibacterial compositions.

### Brief Description of the Drawings

Fig. 1 illustrates the cytotoxicity of Viniferin and grape callus culture extract (Sample A) on MNT-1 cells. Sample A was diluted and treated on wells for comparison with Viniferin based on the epsilon-Viniferin content.
Fig. 2 illustrates the cytotoxicity of Viniferin and grape callus culture extract (Sample A) on Raw 264.7 cells. Sample A was diluted and treated on wells for comparison with Viniferin based on the epsilon-Viniferin content.
Fig. 3 shows the antioxidant effect of grape callus culture extract (Sample A) and its extract (Sample a) by conducting DPPH analysis.
Fig. 4 illustrates the minimum inhibitory concentration of grape callus culture extract (Sample A and Sample C) on three types of *P. acnes* after 72 hours of treatment.
Fig. 5 illustrates the minimum bacteriostatic concentration of grape callus culture extract (Sample A and Sample C) on three types of *P. acnes* after 72 hours of treatment.
Fig. 6 illustrates the minimum bacteriostatic concentration of grape callus culture extract (Sample A and Sample C) on three types of *P. acnes* after 48 hours of treatment.
Fig. 7 demonstrates the antibacterial effect of grape callus culture extract's extract (Sample a) using the agar diffusion method.

Hereinafter, the present invention will be described in detail.

The present invention provides an antioxidant and antibacterial composition containing one or more selected from a group consisting of grape callus, callus lysates, callus culture solution and callus extract as active ingredients.

In the present invention, the callus refers to irregular tissues or cell masses mostly composed of parenchyma cells that have lost the ability for normal organ formation or tissue differentiation, and it is a specialized tissue or cell mass that forms when tissues are excised from a plant and cultured on growth regulator-containing media for tissue culture, or when wounds are inflicted on a plant or growth regulators are applied to plant explants.

Furthermore, the lysate refers to cell lysates obtained by crushing cells using chemical methods such as common detergents or physical methods such as French Press, according to generally known methods in the art, without specific limitations.

Additionally, the culture extract refers to the medium (conditioned medium) obtained by culturing cells using conventional methods in the art and removing the pellet by physical methods such as centrifugation or filtration after culture, according to generally known methods in the art, without specific limitations.

The extract refers to the extraction of callus itself or callus powder, callus lysate, or callus culture solution by commonly known methods in the art, which can be manufactured according to generally known methods in the art, and such methods are not specifically limited. For example, there are methods such as cold maceration, hot water extraction, ultrasound extraction, reflux cooling extraction, etc. Commonly available solvents can be used as extraction solvents, such as water, anhydrous or hydrous lower alcohols with 1-4 carbons, acetone, ethyl acetate, butyl acetate, and 1,3-butylene glycol.

On the other hand, the grape callus and its culture solution are preferably prepared as follows, but are not limited thereto.
1) inducing callus from grape tissue; and
2) culturing the induced grape callus by adding an active inducer and a solubilizing agent.

The grape in step 1) may be selected from any one or more parts selected from a group consisting of flowers, leaves, stems, branches, fruits, and roots of the grape, and more specifically a flower of the grape, wherein the grape variety is Cheongsu, Hongdan, Tamnara, Hongisul, Heukguseul, Heukboseok, Suok, Dunuri, Tamgeumchu, Hongaram, Narsha, Hongsodam, Alden, Campbell Early, Delaware, Dutchess, Golden Muscat, Himrod Seedless, Schuyler, Beniizu, Kyoho, Pione, Super Hamburg, Tano Red, Muscat Hamburg, and Seibel 9110, among others, but are not limited thereto.

In step 1), the induction of callus is achieved by culturing tissue or its explants from the plant on tissue culture media. Various basic plant media such as MS (Murashige and Skoog, 1962) medium, N6 (Chu et al., 1975), B5 (Gamborg et al., 1968), NN (Nitsch and Nitsch, 1967) medium, WHITE medium, among others, can be used as the tissue culture media. Additionally, it is desirable to use tissue culture media containing plant growth regulators. The plant growth regulators used herein may include substances known in the art, but preferably include auxins such as 2,4-D (2,4-dichlorophenoxyacetic acid), 2,4,5-T (2,4,5-trichlorophenoxyacetic acid), Dicamba (2-methoxy-3,6-dichlorobenzoic acid), IAA (indole-3-acetic acid), IBA (indole-3-butyric acid), MCPA (2-methyl-4-chlorophenoxyacetic acid), NAA (1-naphthylacetic acid), NOA (2-naphthyloxyacetic acid), Picloram (4-amino-2,5,6-trichloropicolinic acid) and cytokinins such as BAP (6-benzylaminopurine), 2iP (N6-(2-isopentyl)adenine), Kinetin (6-furfurylaminopurine), Thidiazuron (1-phenyl-3-(1,2,3-thiadiazol-5-yl)urea), Zeatin (4-hydroxy-3-methyl-trans-2-butenylaminopurine), and their derivatives. In addition to these, alkaloids, terpenoids, carotenoids, phenolic compounds, and natural extracts that affect cell differentiation and proliferation can also be added to the tissue culture media. Preferably, IAA, NAA, 2,4-D, and kinetin can be added, and more preferably, 0.05-0.2 mg/L IAA, 0.05-0.2 mg/L NAA, 1-2 mg/L 2,4-D, and 0.2-0.3 mg/L kinetin, but is not limited thereto.

In step 2), the active inducer may be methyl jasmonic acid, SA or plagelin 22, more preferably methyl jasmonic acid, but is not limited thereto. In addition, the solubilizing agent may be cyclodextrin, methylcyclodextrin or stevioside, more preferably stevioside, but is not limited thereto. The methyl jasmonic acid and stevioside may preferably be added at concentrations of 50 ~ 350 µM and 40 ~ 60 mM, respectively, and more preferably at concentrations of 100 µM and 50 mM, respectively, but are not limited thereto.

Furthermore, the composition preferably exhibits antibacterial activity against one or more strains selected from *Propionibacterium acnes, Staphylococcus epidermis, Staphylococcus aureus, Staphylococcus saprophyticus, Pseudomonas aeruginosa, Enterococcus faecalis, Actinomyces baumannii, Bacillus cereus, Bacillus subtilis* subsp. spizizenii, *Klebsiella pneumonia, Klebsiella oxytoca,* and *Micrococcus luteus,* with significant bacteriostatic and bactericidal activity against *Propionibacterium acnes,* and thus, the composition is preferably used as a pharmaceutical composition for the prevention and treatment of acne.

Additionally, the present invention provides a use for an antioxidant and antibacterial composition comprising one or more selected from grape callus, its lysate, its culture solution and its extract as a pharmaceutical composition for the prevention or treatment of acne.

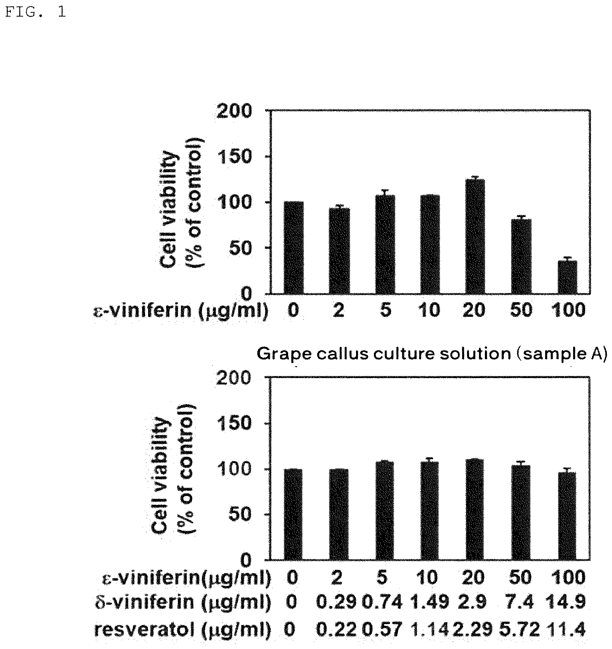

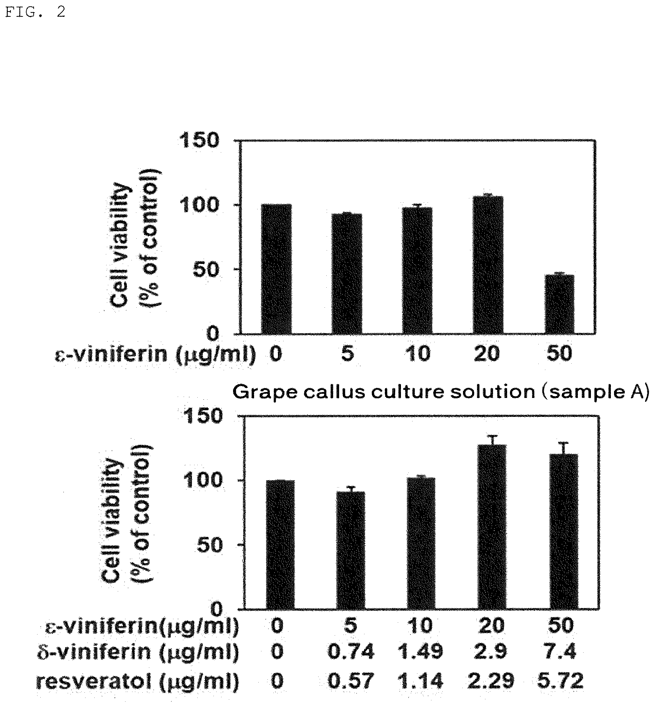

In a specific embodiment of the present invention, after inducing grape callus, the inventors prepared the culture solution and extract thereof, and confirmed cytotoxicity, and found that epsilon-Viniferin was not cytotoxic only within 20 µg/ml, but the grape callus culture solution did not show cytotoxicity even if epsilon-Viniferin was contained in 100 ug/ml (see FIGS. 1 and 2).

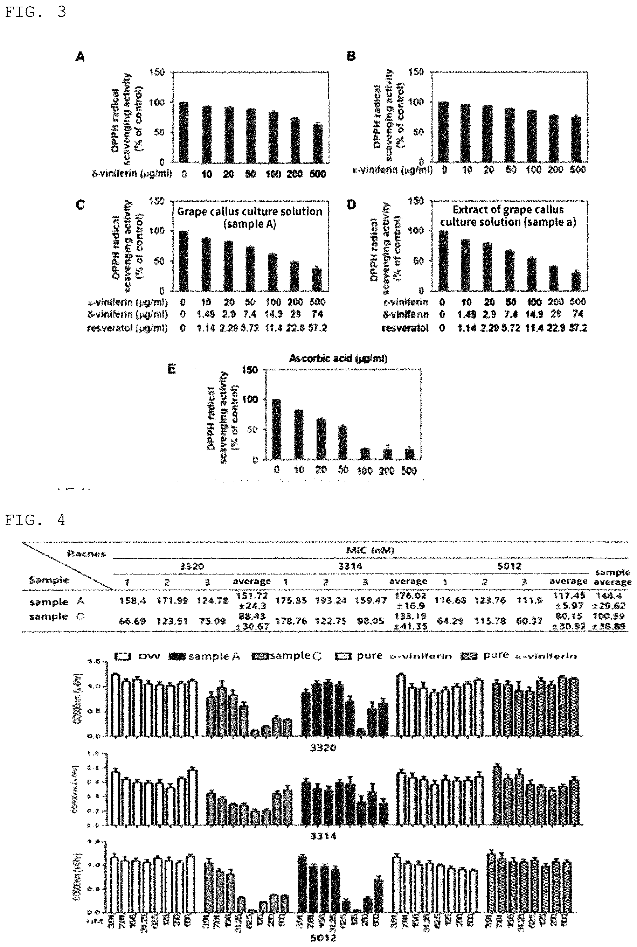

In addition, the inventors performed DPPH analysis to confirm the antioxidant effect of grape callus culture, and results showed that epsilon-Viniferin and delta-Viniferin at 500 µg / ml eliminated radicals by approximately 25% compared to the control group, and in contrast, grape callus culture solution (Sample A) and its extract (Sample a) exhibited radical elimination of over 60% at 500 µg / ml (based on epsilon-Viniferin content)(see FIG. 3).

Furthermore, the inventors confirmed that the antibacterial effect of grape callus culture solution, the grape callus culture solution of the present invention shows a significant bactericidal and bacteriostatic effect on acne bacteria, particularly demonstrating remarkable antibacterial effects compared to pure viniferin (see FIGS. 4 to 7).

Therefore, grape callus, its culture solution, and its extract of the present invention exhibit no cytotoxicity, significant antioxidant effects, and remarkable antibacterial effects against acne bacteria, so that they can be effectively utilized as active ingredients in antioxidant and antibacterial compositions.

Pharmaceutical compositions according to the present invention may contain pharmaceutically acceptable carriers in addition to active ingredients. Pharmaceutically acceptable carriers included in the pharmaceutical compositions of the present invention are those commonly used in formulations, such as carbohydrate compounds (e.g., lactose, amylose, dextrose, sucrose, sorbitol, mannitol, starch, cellulose, etc.), acacia gum, calcium phosphate, alginate, gelatin, calcium silicate, microcrystalline cellulose, polyvinylpyrrolidone, cellulose, water, syrups, saline solutions, alcohol, arabic gum, vegetable oils (e.g., corn oil, cottonseed oil, soybean oil, olive oil, coconut oil), polyethylene glycol, methyl cellulose, methylhydroxy benzoate, propylhydroxy benzoate, activated charcoal, magnesium stearate, and mineral oils, but are not limited thereto.

The pharmaceutical compositions of the present invention may further include lubricants, moisturizers, sweetening agents, flavoring agents, emulsifiers, suspending agents, preservatives, etc., in addition to the above-mentioned ingredients. Suitable pharmaceutically acceptable carriers and formulations are detailed in Remington's Pharmaceutical Sciences (19th ed., 1995).

The pharmaceutical compositions of the present invention can be administered via various routes such as oral or non-oral routes, and all modes of administration are contemplated, and for example, administration may be by oral, rectal, or intravenous, intramuscular, subcutaneous injection. In this case, in the non-oral route, particularly dermal administration is preferred, and local administration with topical application is the most preferable.

Suitable dosages of the pharmaceutical compositions of the present invention may be variously prescribed by factors such as formulation method, mode of administration, patient age, weight, sex, pathological condition, food, administration time, route of administration, excretion rate and response sensitivity. The oral dose of the pharmaceutical composition of the present invention is in the range of 0.001-100 mg / kg (body weight) in an adult standard. In addition, in the case of external use, it can be applied once to 5 times a day in an amount of 1.0 to 3.0 mL for adults and continued for more than 1 month. However, the dosage is not limited to the scope of the present invention.

The pharmaceutical compositions of the present invention may be formulated in a unit dose form or by formulation using pharmaceutically acceptable carriers and/or excipients according to a method that can be easily practiced by one skilled in the art to which the invention belongs. In this case, the formulation may be in the form of a solution, suspension, syrup or emulsion in an oil or aqueous medium, or may be in the form of tablets, powders, granules, capsules, or lozenges, with the addition of dispersants or stabilizers as needed.

In addition, the present invention provides cosmetic composition for antioxidative and antibacterial purposes, comprising one or more selected from a group consisting of grape callus, callus lysate, callus culture solution and callus extract.

In addition, the present invention provides a use of a composition containing as an active ingredient any one or more selected from a group consisting of grape callus, callus lysate, callus culture solution and callus extract for use as cosmetic compositions for acne prevention and improvement.

The effects of grape callus, callus lysates, callus culture solution and callus extract, as well as acne, are as described for the antioxidant and antibacterial compositions above. Specific details are herein incorporated by reference, and the following provides specific descriptions unique to cosmetic compositions.

The cosmetic composition according to the present invention may additionally include components commonly used in the cosmetic composition in addition to the active ingredient. For example, it may include antioxidants, stabilizers, solubilizing agents, conventional supplements such as vitamins, pigments and fragrances, as well as excipients.

The cosmetic compositions of the present invention may be prepared in any formulation commonly made in the art, for example, solutions, suspensions, emulsions, pastes, gels, creams, lotions, powders, soaps, surfactant-containing cleansing, oils, powder foundations, emulsion foundations, wax foundations and sprays, and the like, but are not limited thereto. More specifically, it may be prepared in formulations such as nourishing creams, astringent lotions, flexible lotions, lotions, essences, nourishing gels or massage creams, and the like.

In the case of formulations as paste, cream or gel, the cosmetic compositions may contain animal oil, vegetable oil, wax, paraffin, starch, tracanth, cellulose derivatives, polyethylene glycol, silicon, bentonite, silica, talc or zinc oxide, and the like as carrier components.

In the case of formulations as a powder or a spray, the cosmetic compositions may contain lactose, talc, silica, aluminum hydroxide, calcium silicate or polyamide powder as the carrier component, and in particular, in the case of a spray, it may additionally include a propellant such as chlorofluorohydrocarbon, propane/butane or dimethyl ether.

In the case of formulations as a solution or an emulsion, the cosmetic compositions may contain a solvent, a solubilizing agent or an emulsifying agent as a carrier component, such as water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or sorbitan fatty acid ester.

In the case of formulations as a suspension, the cosmetic composition may contain water, ethanol, or propylene glycol as liquid diluents, ethoxylated stearyl alcohol, polyoxyethylene sorbitol esters, and polyoxyethylene sorbitan esters as suspending agents, microcrystalline cellulose, aluminum hydroxide, bentonite, agar, or tragacanth as viscosity-increasing agents.

In formulations such as surfactant-containing cleansers, the cosmetic compositions may contain excipients such as aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamidobetaine, aliphatic alcohol, fatty acid glycerides, fatty acid diethanolamide, vegetable oil, lanolin derivative or ethoxylated glycerol fatty acid ester, and the like.

Additionally, the present invention provides antioxidant and antibacterial health supplements containing one or more selected from grape callus, callus lysate, callus culture solution, and callus extract as active ingredients.

The effects of grape callus, callus lysate, callus culture solution, and callus extract are as described for the antioxidant and antibacterial compositions, and specific details are incorporated from the aforementioned description. Hereafter, only the composition specific to the health food composition will be described.

The health food according to the present invention may include ingredients commonly added during food manufacturing in addition to the active ingredient. For example, they may contain proteins, carbohydrates, fats, nutrients, flavorings and flavors. Examples of carbohydrates described above include monosaccharides such as glucose, fructose, etc.; disaccharides such as maltose, sucrose, oligosaccharides, etc.; and polysaccharides, such as conventional sugars such as dextrin, cyclodextrin, and the like, and sugar alcohols such as xylitol, sorbitol, erythritol. As flavoring agents, natural flavors [taumatin, stevia extracts (eg, rebaudioside A, glycyrrhizine, etc.) and synthetic flavors (saccharin, aspartame, etc.)] can be used.

The mixed amount of the active ingredient can be suitably determined according to the purpose of its use. In general, the amount of the active ingredient in the health food can be added at a weight of 0.01 to 15 by weight of the total food food, and the health drink composition can be added at the rate of 0.02 to 5 g, preferably 0.3 to 1 g based on 100 ml. However, in the case of long-term intake for health and hygiene purposes or for the purpose of health control, the amount may be less than the above range, and since there is no problem in terms of safety, the active ingredient may also be used in an amount greater than the range.

In the case where the health food of the present invention is prepared with a health drink composition, citric acid, liquid fructose, sugar, glucose, acetic acid, malic acid, and the like may be further included in addition to the active ingredients of the present invention.

In addition, in addition to the above-described ingredients, the health food may contain various nutritional agents, vitamins, electrolytes, flavor agents, colorants, pectic acids and salts thereof, alginic acid and salts thereof, organic acids, protective colloidal thickeners, pH adjusters, stabilizers, preservatives, glycerin, alcohol, carbonated agents used in carbonated drinks, and the like. In addition, the health food of the present invention may contain pulp for the production of natural fruit juice, beverages and vegetable beverages. These components can be used independently or in combination.

In addition, the present invention provides a method for the prevention or improvement of oxidative stress, comprising administering a composition containing at least one active ingredient selected from a group consisting of a pharmaceutically effective amount of grape callus, callus lysate, callus culture solution and callus extract, to a subject.

In addition, the present invention provides a method for the treatment of oxidative stress, comprising administering a composition containing at least one active ingredient selected from a group consisting of a pharmaceutically effective amount of grape callus, callus lysate, callus culture solution and callus extract, to a subject.

In addition, another object of the present invention is to provide a method for the prevention or improvement of bacterial infections, comprising administering a composition containing at least one active ingredient selected from a group consisting of a pharmaceutically effective amount of grape callus, callus lysate, callus culture solution and callus extract, to a subject.

Furthermore, another object of the present invention is to provide a method for the treatment of bacterial infections, comprising administering a composition containing an effective amount of grape callus, callus lysate, callus culture solution, and callus extract, to a subject.

Additionally, the present invention provides a method for the prevention or improvement of acne, comprising administering a composition containing at least one active ingredient selected from a group consisting of a pharmaceutically effective amount of grape callus, callus lysate, callus culture solution and callus extract, to a subject.

Moreover, the present invention provides a method for the treatment of acne, comprising administering a composition containing an effective amount of grape callus, callus lysate, callus culture solution, and callus extract, to a subject.

The grape callus, its lysate, its culture solution and its extract mentioned above, as well as acne, are described in the same manner as described for the antioxidant and antibacterial compositions, and specific descriptions are provided in the foregoing content.

On the other hand, the antioxidant and antibacterial compositions prepared by the method of the present invention exhibit no cytotoxicity, significant antioxidant effects, and antibacterial effects against acne bacteria. Particularly, they demonstrate significant antioxidant and antibacterial effects compared to pure viniferin. Therefore, the antioxidant and antibacterial compositions of the present invention can be effectively utilized for the prevention, improvement, or treatment of acne.

Hereinafter, the present invention will be described in detail by examples and experimental examples.

However, the following examples and experimental examples are only illustrative of the present invention, and the contents of the present invention are not limited by the following embodiments and experimental examples.

### Example < 1> Preparation of grape callus culture solution

### <1-1> Grape callus induction

The grape variety was propagated after inducing callus from grape tissue using Campbell (*Vitis vinifera* L. cv Campbell Early).

Specifically, the flowers of the grapes were cut off, washed with running water, and then immersed in 70% ethanol solution for 1 minute for surface sterilization. Surface sterilization was performed in a glass jar of grape flower tissue in an aseptic work table, commercial lax (about 1% sodium hypochlorite) solution for 15 minutes, and washed three times with sterile distilled water. The tissue that completed the surface sterilization was used to remove the remaining moisture using sterile filter paper and use it for culture. The flower tissue of the grapes that completed the surface sterilization was cut using a scalpel, and then each tissue (petals, anthers, operating table) was placed in the culture medium. The medium used to induce grape callus is shown in Table 1, and a medium with 0.1 mg/LIAA, 0.1 mg/LNAA, 1.5 mg/L 2,4-D and 0.25 mg/L kinetin was used as a growth regulator. The culture conditions were carried out in a 25 °C. constant temperature machine. The white callus formed after culture was subcultured in a solid medium of the same composition at intervals of about 4 weeks.

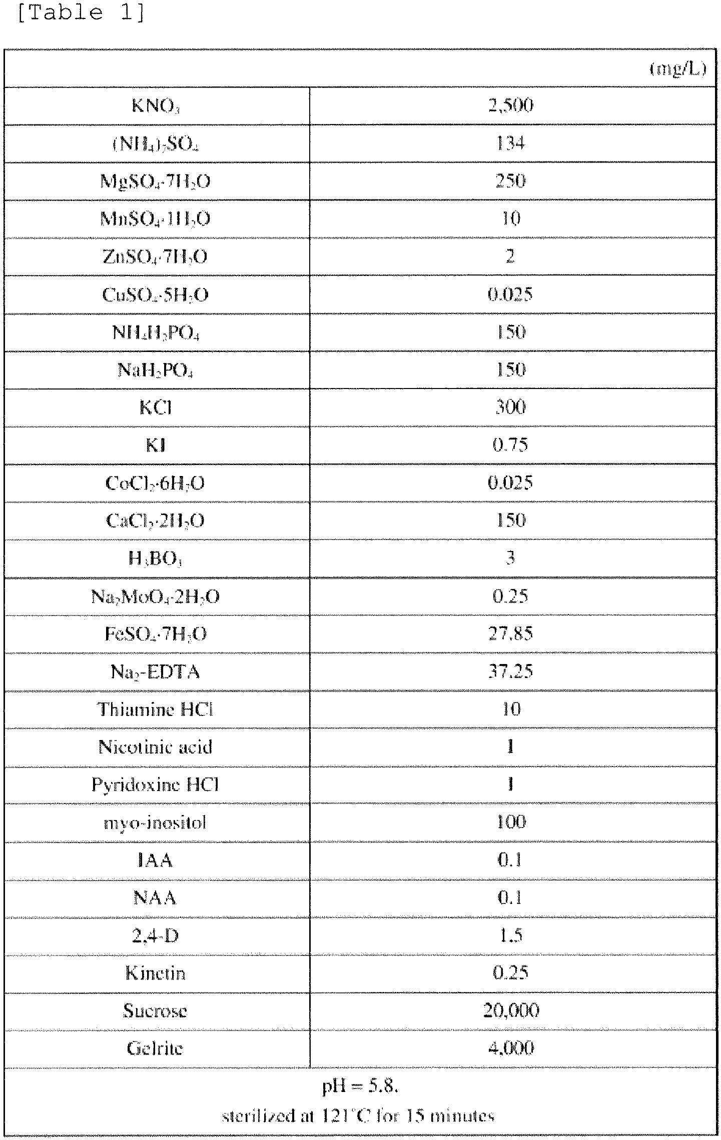

### <1-2> Collection of grape callus culture solution

The stabilized grape callus induced in the above Example <1-1> was cultured to collected the grape callus culture solution.

Specifically, the grape callus derived and stabilized in Example <1-1> was seeded and cultured in three flasks of 100 ml, respectively, in 100 ml of MS1D liquid medium (MS 4.4 g, sucrose 30 g, MES 0.5 g, myo-inositol 0.1 g, thiamine HCl 0.4 mg, 2,4-D 1 mg / L) MS, which is known as a MS(Murashige & Skoog) medium containing 1 mg/L 2,4-D, and incubated at 25 °C and 90 rpm for 14 days. The callus that was cultured and proliferated for 14 days was placed in a pre-sterilized and dried 3L bioreactor, and 1.2 L of MS1D liquid medium was added. After 7 days of incubation, 100 µM MeJA (methyl jasmonate) and 50 mM stevioside were treated together, and thereafter, on the 5th day of culture, the sample was filtered through a strainer and separated into callus and callus culture solution, and grape callus culture solution (sample A) was obtained.

In addition, the results of measuring the content of epsilon-Viniferin, delta-Viniferin and resveratrol present in the prepared grape callus culture solution are shown in Table 2.

On the other hand, the content of epsilon-Viniferin, delta-Viniferin and resveratrol is 20 ml of the grape callus culture (sample A) with the same volume of ethyl acetate, mixed well, and the ethyl acetate layer was collected and ethyl acetate was removed using an evaporator. Finally, it was dissolved in 1 ml of ethanol, then diluted to an appropriate amount and analyzed the content of each epsilon-viniferin, delta-viniferrin, and resveratrol with HPLC.

### <1-3> Preparation of extracts of grape callus cultures

The grape callus culture solution (sample A) obtained in Example <1-2> was extracted with ethyl acetate to prepare an extract (sample a) of the grape callus culture solution.

Specifically, the same volume of ethyl acetate was added to 20 ml of grape callus culture solution (sample A) obtained in Example <1-2> above, and after mixing well, the ethyl acetate layer was collected and ethyl acetate was removed using an evaporator. Finally, it was dissolved in 1 ml of ethanol to prepare an extract (sample a) of callus culture solution.

In addition, the content of epsilon-Viniferin, delta-Viniferin and resveratrol in the extract (sample a) of the callus culture solution was measured in the same way as <1-2> above, and the results are shown in Table 2 below.

### <1-4> Preparation of sample C

In Example <1-2>, the callus that was seeded in a flask and incubated for 14 days was placed in a pre-sterilized and dried flask instead of a bioreactor and then 300 ml of MS1D liquid medium was added. After 7 days of incubation, 100 µM MeJA (methyl jasmonate) and 50 mM stevioside were treated together, and thereafter, grape callus culture solution (named as sample C) was obtained by separating the callus and callus culture solution by filtering the sample through a strainer on the 5th day of culture.

**[Table 2]**

| **Sample** | **Sample composition** (µg/ml) | | |
|---|---|---|---|
| | e-viniferin | resveratol | d-viniferin |
| A | 571.66 | 65.49 | 85.45 |
| a | 11433.12 | 1309.03 | 1709.00 |
| C | 1780 | 850 | 169570 |

### <1-5> Isolation and Purification of Delta-Viniferin

The grape callus culture solution prepared in <1-2> was divided and extracted with water and ethyl acetate, and the ethyl acetate layer obtained was concentrated under reduced pressure. The obtained concentrate (15 g) was separated in one step by a reversed-phase column (10 × 30 cm) using an MPLC instrument. At this time, the eluent solvent was water-acetonitrile and the water-acetonitrile mixed solution was eluted with a lower polarity from 100: 1 to 100% acetonitrile to obtain seven fractions (A-G). Fraction C containing a large amount of the target compound viniferin (TRANCE-d-viniferin (formula 1 below) was concentrated to obtain 1.3 g of concentrate. 1.3 g of the obtained concentrate was performed in reversed-phase column chromatography using a Recylcing LC instrument under water-acetonitrile (2: 2) mixed solvent conditions to obtain 160 mg of viniferrin of 80% or more purity. Gel chromatography (Sephadex LH-20), in which the obtained fraction was eluted with 95% methanol, obtained 12 mg of viniferin of 98% purity. The structure of the obtained compounds was elucidated using ¹H-NMR, ¹³C-NMR, 2D-NMR, DEPT and mass spectrometry.

*trans*-δ-Viniferin: ¹H-NMR (400 MHz, Acetone-*d*₆)*δ* 7.45 (1H, dd, *J* = 8.5,2.0 Hz, H-14b), 7.27 (1H, s, H-10b), 7.25 (2H, d, *J* = 8.5 Hz, H-2(6)a), 7.07 (1H, d, *J* = 16.5 Hz, H-7b), 6.92 (1H, d, *J* = 16.5 Hz, H-8b), 6.88 (1H, d, *J* = 8.1 Hz, H-13b), 6.86 (2H, d, *J* = 8.5 Hz, H-3(5)a), 6.55 (2H, d, *J* = 2.0 Hz, H-2(6)b), 6.29 (1H, t, *J* = 2.0 Hz, 12a), 6.27 (1H, t, *J* = 2.0 Hz, H-4b), 6.21 (2B, d, *J* = 2.0, H-10(14)a), 5.47 (1H, d, *J* = 8.0 Hz, H-7a), 4.48 (1H, d, *J* = 8.0 Hz, H-8a); ¹³C-NMR (100 MHz, Acetone-*d*₆) *δ* 159.7 (C-12b), 158.8 (C-11(13)a), 158.6 (C-3(5)b), 157.5 (C-4a), 144.3 (C-9a), 139.8 (C-1b), 131.6 (C-11b), 131.2 (C-1a), 130.8 (C-9b), 128.2 (C-7b), 127.7 (C-14b), 127.6 (C-2(6)a), 126.3 (C-8b), 123.0 (C-10b), 115.2 (C-3(5)a), 109.2 (C-13b), 106.4 (C-10(14)a), 104.7 (C-2(6)b), 101.8 (C-4b), 101.4 (C-12a), 93.1 (C-7a), 56.9 (C-8a).

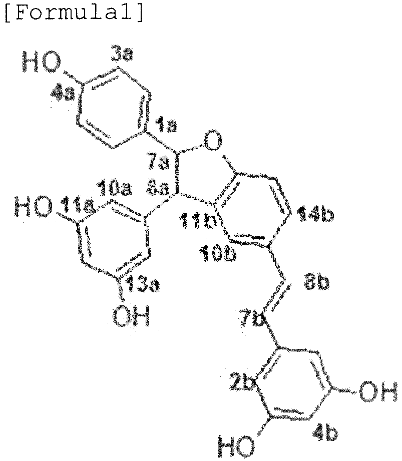

### <1-6> Isolation and Purification of epsilon-Viniferin

The grape callus culture solution prepared in <1-2> was divided and extracted with water and ethyl acetate, and the ethyl acetate layer obtained was concentrated under reduced pressure. The obtained concentrate (10 g) was separated in one step by reversed-phase column (10 × 30 cm) using an MPLC instrument. At this time, the eluent solvent was water-acetonitrile and the water-acetonitrile mixed solution was eluted from 70: 1 to 100% acetonitrile with a lower polarity to obtain five fractions (A-E). Fraction B containing a large amount of the target compound biniferin (trance-ε-viniferin (formula 2 below) was concentrated to obtain 1.5 g of concentrate. 1.5 g of the obtained concentrate was performed in reversed-phase column chromatography using a Recylcing LC instrument under water-acetonitrile (2: 1) mixed solvent conditions to obtain 100 mg of viniferin of 850 or more purity. Gel chromatography (Sephadex LH-20), in which the obtained fraction was eluted with 95% methanol, was performed to obtain 30 mg of viniferin of 98% purity. The structure of the obtained compounds was elucidated using ¹H-NMR, ¹³C-NMR, 2D-NMR, DEPT and mass spectrometry.

trans-ε-viniferin ¹H-NMR (400 MHz, MeOD) *δ* 7.14 (2H, d, *J* = 8.0 Hz, H-2(6)a), 7.03 (2H, d, *J* = 8.0 Hz, H-2(6)b), 6.80 (1H, d, *J* = 13.6 Hz, H-7b), 6.78 (2H, d, *J* = 8.0 Hz, H-3(5)a), 6.65 (2H, d. *J* = 8.0 Hz, H-3(5)b), 6.56 (1H, d, *J* = 13.6 Hz), 6.28 (1H, brs, H-12b), 6.21 (1H, brs, H-12a), 6.18 (2H, brs, H-10a, 14a), 5.37 (1H, d. *J* = 8.0 Hz, H-7a), 4.35 (1H, d, *J* = 4.0 Hz, H-8a); ¹³C-NMR (100 MHz, MeOD) *δ* 161.3 (C-11b), 158.6 (C-11a, 13a), 158.3 (C-13b), 157.1 (C-4a), 156.9 (C-4b), 146.0 (C-9a), 135.6 (C-9b), 132.5 (C-1a), 129.1 (C-1b, 7b), 127.5 (C-2(6)b), 127.0 (C-2(6)a), 122.4 (C-8b), 118.8 (C-10b), 115.1 (C-3(5)b), 115.1 (C-3(5)a), 106.3 (C-14a, 10a), 103.1 (C-14h), 101.0(C-12a), 95.6 (C-12b), 93.5 (C-7a), 56.9 (C-8a).

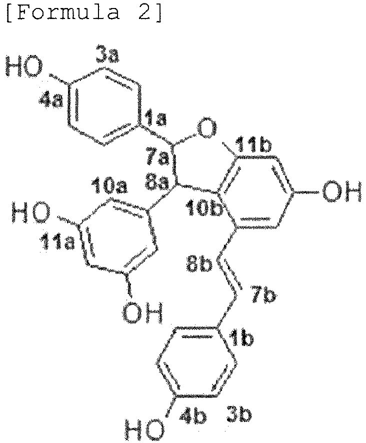

### <Experimental Example 1> Confirmation of cytotoxicity of grape callus culture solution

MTT assay was performed using MNT-1, Raw264.7 cells to confirm the cytotoxicity of grape callus cultures, epsilon-Viniferin and delta-Viniferin.

Specifically, Raw264.7 (murine macrophage) was incubated at 37 °C, 5% CO₂ in DMEM (High glucose) medium with 10% FBS and 1% gentamicine added, and MNT-1 (human melanoma cell) was incubated at 37 °C, 5% CO₂ in DMEM/MEM (High glucose) medium with 20% FBS and 1% gentamicine added. MNT-1 (10⁴ cell/well) and Raw264.7 (2×10⁴ cell/well) were each incubated in 96 wells, the samples were diluted with medium and treated in each well. After 24 hours, the MTT solution was treated at a concentration of 100 ug/mL and reacted at 37 °C, 5% CO₂ incubator for 1 hour. All cultures were then removed, 100 µl of acid-isopropanol was added to each well, and absorbance was measured with an ELISA reader at 570 nm. In addition, the sample was diluted based on epsilon-Viniferin content and treated in a well for comparison with Viniferin.

As a result, as shown in FIGS. 1 and 2, epsilon-Viniferin exhibited no cytotoxicity only within 20 pg/ml, but the grape callus culture solution (sample A) did not show cytotoxicity even when epsilon-Viniferin was present at concentrations of up to 100 µg/ml (FIGS. 1 and 2).

### <Experimental Example 2> Confirmation of antioxidant effect of grape callus culture solution

DPPH analysis was performed to confirm the antioxidant effect of grape callus culture solution.

Specifically, 0.2 mM DPPH (1,1-Diphenyl-2-picryhydrazyl) solution was prepared using 80% methanol, and then 180 µl of DPPH and 20 µl of the sample were well mixed and reacted at room temperature for 10 minutes, and then absorbance was measured with an ELISA reader at 517 nm. On the other hand, the sample was diluted based on epsilon-Viniferin content and treated in a well for comparison with Viniferin. In addition, the control method used DPPH reagent in the dilution solvent of the sample instead of the sample. Ascorbic acid was treated as a positive control.

As a result, as shown in FIG. 3, epsilon-Viniferin and delta-Viniferin exhibited approximately 25% radical scavenging activity at a concentration of 500 pg/ml compared to the control group, but it was observed that in the case of grape callus culture solution (sample A) and the extract of grape callus culture (sample a) at a concentration of 500 ng/ml (based on epsilon-Viniferin content), radical scavenging activity was confirmed to be over 60% (Figure 3).

### <Experimental Example 3> Confirmation of antibacterial effect of grape callus culture solution

### <3-1> Culture of acne bacteria

To confirm the antibacterial effect of grape callus culture solution on acne bacteria, *Propionibacterium acnes* (KCTC 3314, KCTC 3320, KCTC 5012) was cultured in RCM (Reinforced Clostridial Medium) at 37 °C, anaerobic conditions.

### <3-2> Check MIC (minimum inhibitory concentration) for acne bacteria

MIC is an index for measuring bacteriostatic characteristics of antibacterial substances, and after diluting the grape callus culture solution of the present invention to a concentration between 500 nM - 3.91 nM, the minimum concentration that inhibits the growth of three kinds of *P. acnes* was examined.

Specifically, three kinds of *P. acnes* cultured in <3-1> were incubated in an incubator at 37 °C for 72 hours under anaerobic conditions in 5 ml of RCM liquid medium. In addition, a master plate was prepared at 8 concentrations by diluting 1/2 from the highest concentration to DW in 96 well plates. 50 µl of each diluted sample was then transferred to another 96-well plate. The three *P. acnes* were diluted to 1×10⁶ cells/50 ul (in 2xRCM) and 50 µl (1×10⁶ cells) were transferred to a 96 well plate containing a sample each.

To measure the MIC, 0 hour OD600 nm was measured with a Microplate Reader. Then, after incubating the 96-well plate in 72-hour 37 °C, incubation under anaerobic conditions, measuring OD600 nm for 72 hours with a Microplate Reader, subtracting the 0-hour OD value from the 72-hour OD value, the growth of three *P. acnes* according to control and sample concentration was compared, and antibacterial activity was confirmed through OD value comparison.

As a result, as shown in FIG. 4, grape callus culture solution(sample A) inhibits growth at an average concentration of 151.72 nM, 176.02 nM, and 117.45 nM for *P. acnes* 3320, 3314, and 5012, respectively, and grape callus culture solutionwith high δ-viniferin content (sample C) inhibits growth at an average concentration of 88.43 nM, 133.19 nM, and 80.15 nM for *P. acnes* 3320, 3314, and 5012, respectively . On the other hand, it was confirmed that pure ε-viniferin and pure δ-viniferin did not show growth inhibition for *P. acnes* 3 species.

Therefore, it was confirmed that the grape callus culture solution of the present invention exhibits remarkable bacteriostatic activity compared to pure viniferin.

### <3-3> MBC (minimum bactericidal concentration) analysis for acne bacteria

The Minimum Bactericidal Concentration (MBC) is an indicator measuring the bactericidal properties of an antibacterial substance, and the grape callus culture extract of the present invention was diluted to concentrations ranging 500 nM ~ 3.91 nM, and the minimum concentration inhibiting the growth of three strains of *P. acnes* was determined.

Specifically, the minimum bactericidal concentration was measured using a plate measuring the MIC of <3-1>.

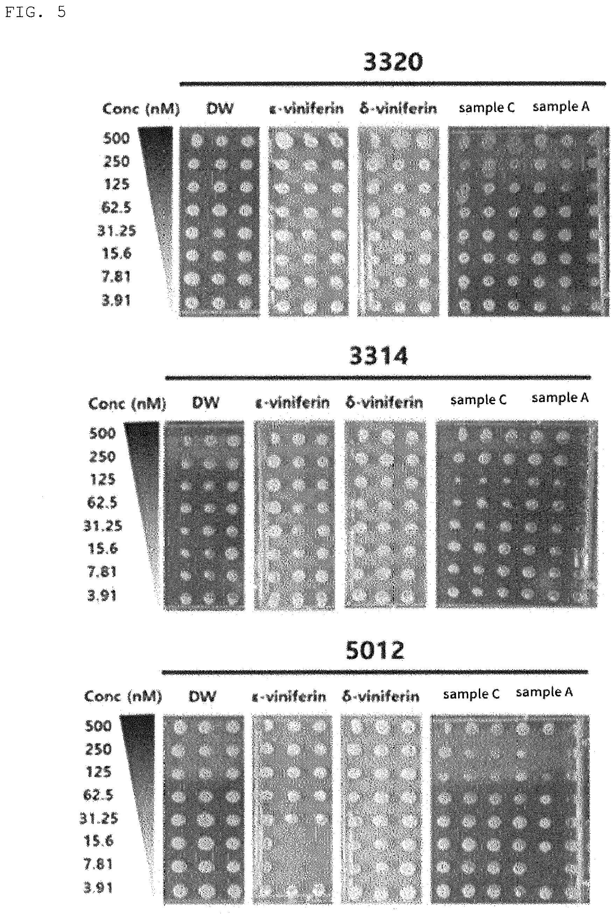

As a result of measuring the 72-hour sterilization of the antibacterial material through MBC, as shown in FIG. 5, the grape callus culture solution (sample A) had the highest growth inhibition effect at 125 nM for *P. acnes* 3320, 3314, and 5012, respectively. In addition, grape callus culture solution (sample C) showed the most growth inhibition at concentrations of 125 nM for *P. acnes* 3320 and 5012 and 62.5 nM for *P. acnes* 3314.

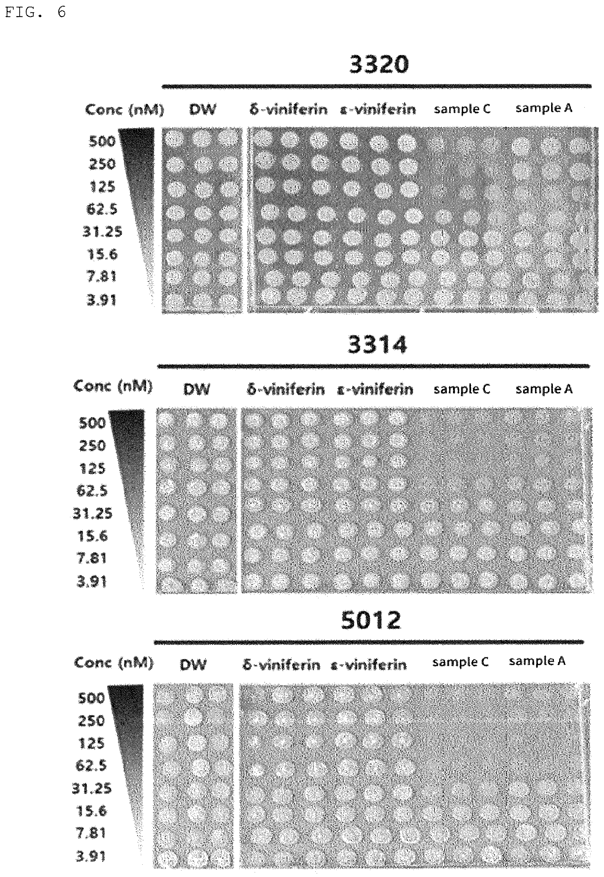

In addition, as shown in FIG. 6, as a result of measuring 48 hr sterilization of the antibacterial material through MBC, the grape callus culture solution (sample A) had the highest growth inhibitory effect at 125 nM for *P. acnes* 3320 and 3314, respectively, and the highest growth inhibitory effect at 62.5 nM for *P. acnes* 5012 and 125 nM for *P. acnes* 3314.

In addition, it was confirmed that pure ε-viniferin and pure δ-viniferin did not show growth inhibition on *P. acnes 3* at both 48 hours and 72 hours.

Thus, it was confirmed that the grape callus culture solution of the present invention exhibits remarkable bactericidal activity compared to pure viniferin.

### <3-4> Confirmation of antibacterial effect through disc diffusion test

The disc diffusion test is an antibacterial agent sensitivity test method of bacteria, and if there is an antibacterial effect, a clear zone is formed around the disk paper, and the antibacterial ability can be measured through the degree of clear zone formation.

Specifically, *P. acnes* 3320 was incubated in 5 ml of RCM liquid medium in an incubator at 37 °C for 72 hours under anaerobic conditions. Then, the bacteria diluted with 5×10⁵ ~ 1×10⁶ were moistened with a cotton swab and evenly smeared on RCM agar medium. The disk paper was placed on an agar plate, the disk paper was moistened with a sample of the stock solution, and then incubated in a 72-hour 37 °C incubator under anaerobic conditions, and then the clear zone size was measured using a Vernier Caliper.

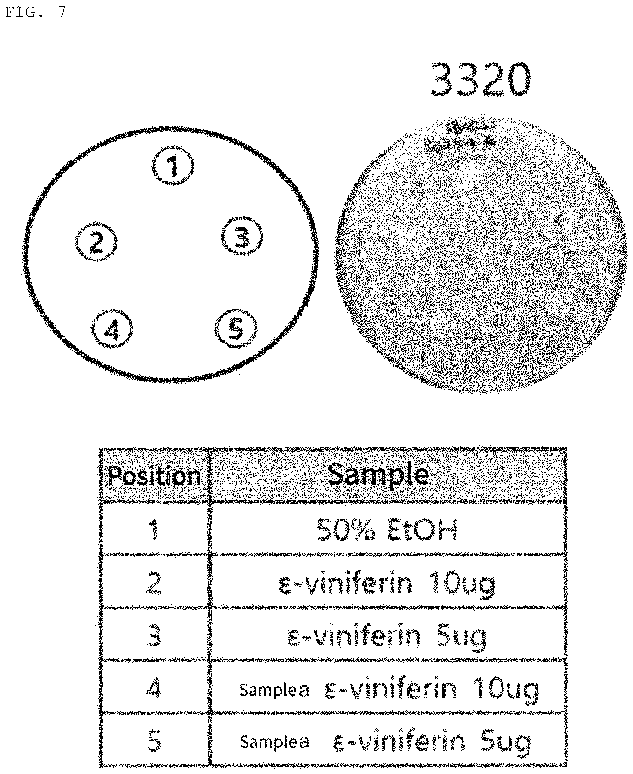

As a result, when the extract (sample a) of the grape callus culture solution was treated with 5 µg and 10 µg based on ε-viniferin as shown in FIGS. 7 and Table 3, antibacterial properties were found for two *P. acnes* (*P. acnes* 3320, 5012), and specifically, an 8 mm clear zone appeared in the control 50% EtOH treatment group, but when the extract (sample a) of the grape callus culture solution of the present invention was treated with 5 µg and 10 µg based on ε-viniferin, a clear zone of 8 mm or more appeared. It was confirmed that the sample a shows antibacterial activity against acne bacteria.

**[Table 3]**

| **Sample** | | clear zone diameter(mm) | | |
|---|---|---|---|---|
| | | 1 | 2 | Average |
| **Pure** | ε-viniferin 10µg | 0 | 0 | 0 |
| | ε-viniferin 5µg | 0 | 0 | 0 |
| **Sample a** | ε-viniferin 10µg | 10.25±0.35 | 10±0 | 10.13±0.25 |
| | ε-viniferin 5µg | 9.5±0 | 9t0 | 9.25±0.28 |

### Industrial Applicability

The present invention relates to an antibacterial and antioxidant composition containing a grape callus culture solution including epsilon(ε)-Viniferin, delta(δ)-Viniferin and resveratrol as an active ingredient, specifically, the grape callus, its culture solution and its extract exhibit no cytotoxicity, and show significant antioxidant effect and antibacterial effect on acne bacteria, and have a significant antioxidant effect and antibacterial effect compared to pure viniferin, making them useful for various applications.

## Claims

1. An antibacterial pharmaceutical composition containing one or more active ingredients selected from a group consisting of grape callus, its lysate, its culture solution and its extract.

2. The antibacterial pharmaceutical composition of claim 1, wherein the grape callus is **characterized by** deriving from any one or more sites selected from a group consisting of flowers, leaves, stems, branches, fruits and roots of grapes.

3. The antibacterial pharmaceutical composition of claim 1, wherein the grape callus is **characterized by** culturing in a medium supplemented with an active inducer and a solubilizing agent.

4. The antibacterial pharmaceutical composition of claim 3, wherein the active inducer and the solubilizing agent are **characterized by** methyl jasmonate and stevioside, respectively.

5. The antibacterial pharmaceutical composition of claim 1, wherein the composition is **characterized by** having an antibacterial activity against one or more strains selected from a group consisting of *Propionibacterium acnes, Staphylococcus epidermis, Staphylococcus aureus, Staphylococcus saprophyticus, Pseudomonas aeruginosa, Enterococcus faecalis, Actinomyces baumannii, Bacillus cereus, Bacillus subtilis subsp. spizizenii, Klebsiella pneumonia, Klebsiella oxytoca* and *Micrococcus luteus.*

6. The antibacterial pharmaceutical composition of claim 1, wherein the composition is **characterized by** having a bacteriostatic and bactericidal activity.

7. A pharmaceutical composition for the prevention and treatment of acne containing one or more active ingredients selected from a group consisting of grape callus, its lysate, its culture solution and its extract.

8. The pharmaceutical composition of claim 7, wherein the composition is **characterized by** having an antibacterial activity against *Propionibacterium acnes.*

9. The pharmaceutical composition of claim 7, wherein the composition is **characterized by** having a bacteriostatic and bactericidal activity against *Propionibacterium acnes.*

10. An antibacterial cosmetic composition containing one or more active ingredients selected from a group consisting of grape callus, its lysate, its culture solution and its extract.

11. A cosmetic composition for the prevention and improvement of acne containing one or more active ingredients selected from a group consisting of grape callus, its lysate, its culture solution and its extract.

12. An antioxidant composition containing one or more active ingredients selected from a group consisting of grape callus, its lysate, its culture solution and its extract.

13. An antioxidant and antibacterial health food containing one or more active ingredients selected from a group consisting of grape callus, its lysate, its culture solution and its extract.

14. A method for the prevention or improvement of oxidative stress, comprising the process of administering pharmaceutically effective amounts of composition containing one or more active ingredients selected from a group consisting of grape callus, its lysate, its culture solution and its extract, to a subject.

15. A method for the treatment of oxidative stress, comprising the process of administering pharmaceutically effective amounts of composition containing one or more active ingredients selected from a group consisting of grape callus, its lysate, its culture solution and its extract, to a subject.

16. A method for the prevention or improvement of bacterial infection, comprising the process of administering pharmaceutically effective amounts of composition containing one or more active ingredients selected from a group consisting of grape callus, its lysate, its culture solution and its extract, to a subject.

17. A method for the treatment of bacterial infection, comprising the process of administering pharmaceutically effective amounts of composition comprising one or more active ingredients selected from a group consisting of grape callus, its lysate, its culture solution and its extract, to a subject.

18. A method for the prevention or improvement of acne, comprising the process of administering pharmaceutically effective amounts of composition containing one or more active ingredients selected from a group consisting of grape callus, its lysate, its culture solution and its extract, to a subject.

19. A method for the treatment of acne, comprising the process of administering pharmaceutically effective amounts of composition containing one or more active ingredients selected from a group consisting of grape callus, its lysate, its culture solution and its extract, to a subject.

20. A use of a composition comprising one or more active ingredients selected from a group consisting of grape callus, its lysate, its culture solution and its extract for use of a composition for the prevention, improvement or treatment of oxidative stress.

21. A use of a composition comprising one or more active ingredients selected from a group consisting of grape callus, its lysate, its culture solution and its extract for use of a composition for the prevention, improvement or treatment of bacterial infection.

22. A use of a composition comprising one or more active ingredients selected from a group consisting of grape callus, its lysate, its culture solution and its extract for use of a pharmaceutical composition for the prevention or treatment of acne.

23. A use of a composition comprising one or more active ingredients selected from a group consisting of grape callus, its lysate, its culture solution and its extract for use of a cosmetic composition for the prevention and improvement of acne.
